# EUROPÄISCHE PATENTSCHRIFT

(11) **EP 3 213 717 B1**
(45) Veröffentlichungstag und Bekanntmachung des Hinweises auf die Patenterteilung: **19.09.2018**
(21) Anmeldenummer: 17158339.6
(22) Anmeldetag: 28.02.2017
(51) Int. Cl.: A61F 2/24, A61F 2/966

(54) **EINFÜHRKATHETER UND KATHETERANORDNUNG**
DELIVERY CATHETER AND CATHETER ARRANGEMENT
CATHÉTER DE LIVRAISON ET ARRANGEMENT DE CATHÉTER

(30) Priorität: 03.03.2016 EP 16158376
(43) Veröffentlichungstag der Anmeldung: 06.09.2017
(73) Patentinhaber: Biotronik AG, 8180 Bülach (CH)
(72) Erfinder: Keller, Mark, 5000 Aarau (CH); Hepke, Markus, 8006 Zürich (CH); Maspoli, Peter, 8180 Bülach (CH)
(74) Vertreter: Galander, Marcus

(56) Entgegenhaltungen:
- WO-A1-2015/031024
- US-A1- 2013 110 223
- US-A1- 2014 277 574

## Beschreibung

Die Erfindung betrifft einen Einführkatheter zum Implantieren eines kardiovaskulären Implantats, insbesondere einer Herzklappenprothese, mit einem ersten, inneren Katheterschaft, welcher innerhalb von mindestens einem zweiten, äußeren Katheterschaft angeordnet ist, wobei das Implantat im distalen Bereich des Einführkatheters auf dem ersten, inneren Katheterschaft angeordnet ist und wobei ein Abschnitt nahe dem distalen Ende des zweiten, äußeren Katheterschafts als Implantatkapsel zur Umhüllung des Implantats während des Einführens ausgebildet ist. Sie betrifft des Weiteren eine mit diesem gebildete Katheteranordnung.

Die Erfindung betrifft einen Einführkatheter zum Implantieren eines selbstexpandierenden Implantates. Derartige selbstexpandierende Implantate sind insbesondere Stents, Herzklappenprothesen, Venenklappenprothesen, Stentsysteme zum Verschließen des linken Herzohres, zum Verschließen peristierender Foramen Ovale, zum Verschluss eines persistierenden Ductus arteriosus, zum Verschluss eines Ventrikelseptumdefektes oder zum Verschluß eines Vorhofseptumdefektes (Okkluder oder Schirmchen) oder selbstexpandierende Filter- oder Protektorsysteme. Die Erfindung eignet sich insbesondere für Einführkatheter für Herzklappenprothesen, die ein selbstexpandierendes Grundgerüst (Herzklappenstent) und eine daran befestigte Klappenanordnung, beispielsweise aus biologischem Gewebe, aufweisen. Die Erfindung wird daher hauptsächlich am Beispiel einer derartigen Herzklappenprothese beschrieben, wie sie beispielsweise als Ersatz einer natürlichen Aortenklappe verwendet werden kann.

Minimal invasive chirurgische Eingriffe gewinnen seit Jahren ständig an Bedeutung und sind beispielsweise zur Behandlung von Stenosen unverzichtbar. In letzter Zeit werden sie auch verstärkt bei der Implantation künstlicher Herzklappen eingesetzt. Geeignete Einführkatheter sind in großer konstruktiver Vielfalt bekannt und Gegenstand ständiger Weiterentwicklung. In den letzten Jahren wurde hier bei der Weiterentwicklung zu Kathetern besonderes Augenmerk geschenkt, die nicht nur das Setzen, sondern auch das Zurückziehen bzw. eine Repositionierung (wenn nötig) von kardiovaskulären Implantaten ermöglichen.

Derartige Einführkatheter bestehen im Wesentlichen aus einem ersten, inneren Katheterschaft, auf dessen distalem Ende das Implantat angeordnet ist. Das Implantat und der erste, innere Katheterschaft sind von einem zweiten, äußeren Katheterschaft umgeben. Der distale Bereich des zweiten äußeren Katheterschafts, der das Implantat umgibt, wird häufig als Implantatkapsel, oder gelegentlich als Katheterhülle bezeichnet. Die Implantatkapsel kann dabei aus dem gleichen Material wie der zweite äußere Katheterschaft oder aus einem anderen Material bestehen, welches mit dem zweiten äußeren Katheterschaft verbunden wird. Hier wird unter der Positionsbezeichnung "proximal" ein näher zum Behandler liegender Teil des Einführkatheters bezeichnet, "distal" bezeichnet entsprechend einen Teil des Einführkatheters, der sich weiter entfernt vom Behandler befindet.

Häufig sind die Implantate (insbesondere der Herzklappenstent) aus einem Formgedächtnismaterial ausgeführt. In diesen Fällen werden die Implantate während des Einführens an den Implantationsort durch die, sie umgebende, Katheterhülle in ihrer komprimierten Form gehalten. Durch Verschieben der Katheterhülle, beispielsweise nach proximal, verschwindet die Rückhaltekraft, die die Katheterhülle auf das Implantat ausübt, und das Implantat expandiert.

Vor kurzem wurden Lösungen vorgeschlagen, die ein Zurückführen ("resheathing" oder "recapturing") eines bereits teilweise freigesetzten Implantats in den Einführkatheter, also speziell in die Implantatkapsel, ermöglichen sollen. Derartige Lösungen werden von den Operateuren stark begrüßt, weil sie Korrekturen während des Einführvorganges ermöglichen und somit helfen, den Implantationsvorgang mit dem bestmöglichen Ergebnis abzuschließen. Speziell in den Fällen, wo das Implantat durch ein Zurückziehen der Katheterhülle nach proximal freigesetzt wird, treten bei der Rückführung des Herzklappenstents in die Implantatkapsel bzw., präziser formuliert, beim Wiederaufstülpen des distalen Katheterendes über den Stent, relativ hohe Reaktionskräfte bzw. hohe lokale Spannungskonzentrationen auf. Diese führen zu komplexen Problemen wie beispielsweise in der Beschreibung des Standes der Technik in der US 2011/0098804 beschrieben und mit dem Versuch einer (teilweisen) Lösung verknüpft ist.

Speziell kann es in Einzelfällen, wie die Erfinder bei entsprechenden praktischen Erprobungen bekannter Systeme festgestellt haben, zu einem funktionellen Versagen der Implantatkapsel beim Zurückführen über das Implantat kommen, und zwar speziell zur Bildung von Knickstellen an der Implantatkapsel, die das Resheathen erschweren oder praktisch oder ganz unmöglich machen.

Der Erfindung liegt daher die Aufgabe der Bereitstellung eines verbesserten Einführkatheters zu Grunde, bei dem insbesondere eine Knickbildung im Bereich der Implantatkapsel weitgehend verhindert und hierdurch ein zuverlässiges und leichtes Resheathen ermöglicht werden soll.

Diese Aufgabe wird durch einen Einführkatheter mit den Merkmalen des Anspruchs 1 gelöst. Zweckmäßige Fortbildungen des Erfindungsgedankens sind Gegenstand der abhängigen Ansprüche. Mit der Erfindung wird weiterhin eine verbesserte Einführkatheteranordnung bereitgestellt.

Neben der oben erwähnten Erkenntnis der Erfinder schließt vorliegende Erfindung den Gedanken ein, im Bereich der Implantatkapsel eine gewissermaßen dynamisch wirksame Versteifung vorzusehen, die - anders als früher vorgeschlagene Lösungen - nicht einfach eine Gesamt-Versteifung der Kapsel durch Erhöhung der Wandstärke oder durch Vorsehen eines besonders dicken inneren Katheterschaftes darstellt. Diese früher vorgeschlagenen Lösungen haben nämlich nach den Feststellungen der Erfinder Nachteile in Bezug auf die Biegeweichheit des Katheters, insbesondere bei aufwändigen Implantatkonstruktionen, wie Herzklappenprothesen. Gleiches gilt für selbstexpandierende Implantate, die in kleineren gebogenen Gefäßen freigesetzt werden sollen.

Erfindungsgemäß ist vorgesehen, dass direkt proximal vom Implantat am ersten, inneren Katheterschaft positionsfest ein biegeweiches distales Stützelement mit einem im wesentlichen kegelstumpfförmigen Abschnitt angebracht ist, der sich in proximaler Richtung verjüngt, und/oder dass am proximalen Ende der Implantatkapsel in den zweiten, äußeren Katheterschaft positionsfest ein biegeweiches proximales Stützelement mit einem im wesentlichen kegelstumpfförmigen Abschnitt eingefügt ist, der sich in proximaler Richtung verjüngt. Die hiermit im Bereich der Implantatkapsel vorgesehenen, jeweils in Bezug auf eines der relativ zueinander verschiebbaren Katheterelemente beweglichen Koni unterdrücken weitgehend die Knick-Neigung bei zusammen auftretenden Biegemomenten und axialen Druckkräften, wie sie beim Resheathen zu beobachten sind. Das bisher beobachtete Knicken, Kollabieren und somit Versagen der distalen äußeren Katheterhülle bzw. Implantatkapsel kann hierdurch weitgehend verhindert werden.

Unter einem kegelstumpfförmigen Abschnitt wird hier jede im Wesentlichen rotationssymmetrische kegelstumpfartige Form verstanden, die sich von einem kleinen Querschnitt zu einem großen Querschnitt ändert. Dies sind zum Einen Kegelstümpfe im streng mathematischen Sinne als auch Kegelstümpfe, wo die Verbindung zwischen den unterschiedlichen Querschnitten von einer Geraden (wie bei einem mathematischen Kegel) abweicht. Dies können insbesondere auch Kegelstümpfe sein, bei deren Schnitt entlang der Längsachse die Außenseiten leicht konkav oder konvex sind.

Unter direkt proximal vom Implantat wird hier eine Anordnung verstanden, wo das Stützelement keinen oder nur ein sehr geringen Abstand vom Implantat hat. Bei Einführkathetern, wo das Implantat an seinem proximalen Ende mit einem Halteelement mit dem ersten inneren Schaft verbunden oder anderweitig befestigt ist, würde sich das Stützelement direkt proximal neben einem derartigen Halteelement befinden.

Das proximale Stützelement verbindet zusätzlich zu seiner Stützfunktion den zweiten äußeren Schaft mit der eigentlichen Implantatkapsel und bildet so einen Übergang vom zweiten äußeren Schaft auf die eigentliche Implantatkapsel.

In einer Ausführung der Erfindung umfasst das proximale Stützelement einen zylindrischen distalen Abschnitt, dessen Durchmesser an den Innendurchmesser der Implantatkapsel und den Außendurchmesser des gecrimpten Implantats angepasst ist, einen zylindrischen proximalen Abschnitt, dessen Außendurchmesser an den Innendurchmesser des zweiten, äußeren Katheterschafts angepasst ist, und einen im wesentlichen kegelstumpfförmigen mittleren Abschnitt, der einen stetigen Übergang zwischen dem distalen und proximalen zylindrischen Abschnitt bildet. Es kann speziell als Spritzguss- oder Drehteil ausgebildet sein, gegebenenfalls aber auch durch partielles Aufweiten aus einem Rohrstück hergestellt sein. Ebenfalls ist die Herstellung mittels Tiefziehen oder Blasformung denkbar. Als Material für das Stützelement sind Metalle, wie Stahl oder Nitinol, oder Kunststoffe zweckmäßig.

In einer weiteren Ausgestaltung weist das proximale Stützelement, insbesondere mindestens im mittleren Abschnitt, eine Vielzahl von Einschnitten auf, die die Biegeweichheit des Stützelementes bewirken. Es ist insbesondere so gestaltet, dass die Einschnitte sich jeweils über einen Teil, bevorzugt den größeren Teil des Umfangs des Stützelementes tangential erstrecken. In dieser Ausführungsform der Erfindung ist das proximale Stützelement als Konus aus einem geschlitzten Metallrohr ausgeführt ("laser cut metal tube").

Bevorzugt bildet das erste proximale Stützelement die Verbindung zwischen dem zweiten äußeren Katheterschaft und der Implantatkapsel oder das proximale Stützelement wird innerhalb der Implantatkapsel an deren proximalen Ende eingesetzt und mit dieser verbunden. In jedem Fall ist das proximale Stützelement derart ausgestaltet, dass die relative Beweglichkeit zwischen dem zweiten äußeren Schaft bzw. Implantatkapsel und dem ersten inneren Katheterschaft gewährleistet ist, das heißt, dass proximale Stützelement weißt ein entsprechendes Innenlumen auf, welches mindestens dem Durchmesser des ersten inneren Katheterschafts entspricht.

In einer weiteren Ausführung ist vorgesehen, dass das distale Stützelement ein distales Ende, dessen Außendurchmesser an den Innendurchmesser der Implantatkapsel und den Außendurchmesser des gecrimpten Implantats angepasst ist, und ein proximales Ende hat, dessen Innendurchmesser an den Außendurchmesser des ersten, inneren Katheterschafts angepasst ist. Des Weiteren ist das distale Stützelement insbesondere als massives oder nicht-massives Kunststoffteil mit einem Innenlumen ausgebildet, dessen Durchmesser an den Innendurchmesser des ersten, inneren Katheterschafts angepasst ist. Aus derzeitiger Sicht bevorzugt ist eine Fertigung des distalen Stützelementes als vom inneren Katheterschaft separates Teil, welches auf den inneren Katheterschaft aufgesteckt und mit diesem verschweißt oder verklebt wird. Grundsätzlich lässt sich das distale Stützelement aber auch einstückig mit dem ersten, inneren Katheterschaft fertigen. Auch das distale Stützelement ist bevorzugt ein Spritz- oder Drehteil und kann optional eine Vielzahl von Einschnitten zur Steigerung der Biegeweichheit aufweisen. Im Prinzip lässt sich das distale Stützelement wie vorangehend für das proximale Stützelement ausgestalten.

In weiteren Ausführungen der Erfindung ist die Implantatkapsel um die Längserstreckung des distalen, falls vorgesehen, und/oder des proximalen Stützelementes, falls vorgesehen, gegenüber der auf die Länge des gecrimpten Implantats bezogene Länge vergrößert. Je nach konkreter Ausführung des Implantats einerseits und des distalen und/oder proximalen Stützelementes andererseits kann eine solche Verlängerung der Implantatkapsel aber auch verzichtbar sein.

In weiteren Ausführungen der Erfindung ist die Dimensionierung des oder jedes Stützelementes so gewählt, dass das Verhältnis zwischen Länge und größtem Durchmesser des Stützelements zwischen 2 und 7, bevorzugt zwischen 3 und 5 und insbesondere bevorzugt zwischen 3,5 und 4,5 beträgt. Des Weiteren, in Kombination mit diesen Dimensionierungsvorschriften oder auch unabhängig hiervon, ist bevorzugt das Verhältnis zwischen der Länge des distalen und/oder des proximalen Stützelementes und der Länge der Implantatkapsel zwischen 0,13 und 0,82, insbesondere zwischen 0,28 und 0,42.

In einer bevorzugten Ausgestaltung mit einer Implantatkapsel mit einem Außendurchmesser von 14 French (4,7 mm) bis 21 French (7 mm) und einer Länge von 60 mm bis 80 mm hat das proximale und/oder distale Stützelement eine Länge von 10 mm bis 50 mm, bevorzugt zwischen 20 mm und 25 mm. Der größte Durchmesser des/der Stützelementes/Stützelemente ist dann abhängig von der verwendeten Wandstärke der Implantatkapsel zwischen 4,5 mm bis 6,5 mm.

Aus derzeitiger Sicht ist eine Ausführung der Erfindung bevorzugt, bei der sowohl ein distales als auch ein proximales Stützelement vorgesehen ist und beide zusammen einen Anteil der Länge der Implantatkapsel von mindestens 0,25 bis 0,85, bevorzugt von 0,45 bis 0,65, stützen. Diese ist zwar aufwändiger als das Vorsehen nur eines Stützelementes, bietet aber weitestgehende Sicherheit gegen ein Knicken und anschließendes Versagen der Implantatkapsel bzw. äußeren Katheterhülle.

In Ausgestaltungen der Erfindung, wo sowohl ein distales und ein proximales Stützelement verwendet werden, werden die beiden Stützelement bevorzugt ineinander passend ausgeführt. Das proximale Stützelement wird dann bevorzugt als Hohlkörper ausgeführt. Im Einführzustand des Katheters wird das Implantat von der Implantatkapsel überdeckt und in seiner komprimierten Form gehalten. Im Einführzustand ist daher bevorzugt das distale Stützelement auf dem Innenschaft zumindest teilweise innerhalb des proximalen Stützelementes angeordnet, welches einen Übergang zwischen dem zweiten äußeren Katheterschaft und der Implantatkapsel bildet. Um ein Verkeilen des distalen Stützelementes im proximalen Stützelement zu verhindern, wird bevorzugt ein Anschlag innerhalb des proximalen Stützelementes angeordnet. Alternativ können die Winkel der konischen Abschnitte des proximalen und distalen Stützelementes unterschiedlich ausgestaltet werden. Beide Konen passen so nicht perfekt ineinander. Alternativ kann ein Verkeilen der Stützelemente auch durch eine entsprechende Oberflächengestaltung oder Oberflächenstruktur verhindert werden (z.B. Oberflächentexturen, Rillen, Noppen etc). Alle drei Ausgestaltungen zur Verhinderung des Verkeilens können beliebig miteinander kombiniert werden.

In einer bevorzugten Ausgestaltung weist der Einführkatheter sowohl ein distales als auch ein proximales Stützelement auf. In dieser Ausgestaltung sind beide Stützelemente bevorzugt als Drehteile aus Edelstahl ausgeführt. Zur Erhöhung der Biegeweichheit sind die Stützelemente mit Einschnitten versehen, die sich über einen Teil, bevorzugt den größten Teil, des Umfangs tangential erstrecken. Das distale Stützelement ist bevorzugt als integrales Bauteil mit dem Implantat-Konnektor ausgeführt.

Vorteile und Zweckmäßigkeiten der Erfindung ergeben sich im Übrigen aus der nachfolgenden Beschreibung eines Ausführungsbeispiels anhand der Figuren. Von diesen zeigen:
- Fig. 1: eine schematische Längsschnittdarstellung des distalen Abschnitts eines Einführkatheters im Zustand des teilweisen Resheathing,
- Fig. 2: eine perspektivische Darstellung einer Ausführungsform des proximalen Stützelements des Einführkatheters nach Fig. 1 und
- Fig. 3: eine perspektivische Darstellung einer Ausführungsform des distalen Stützelementes nach Fig. 1

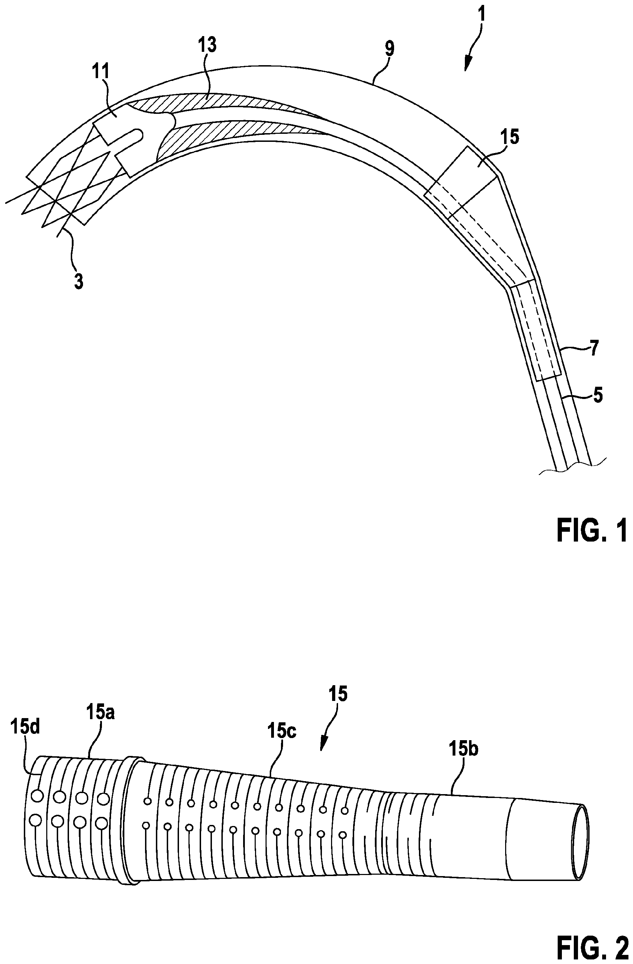

Fig. 1 zeigt schematisch und unmaßstäblich, ohne Beachtung der Wandstärke oder sonstiger konstruktiver Einzelheiten, das distale Ende eines Einführkatheters 1 zur Einführung einer Herzklappenprothese 3, der hier im teilweise in das distale Ende des Einführkatheters 1 zurückgezogenen Zustand (partielles Resheathing) gezeigt ist. Der Einführkatheter 1 umfasst einen ersten, inneren Katheterschaft 5 und einen zweiten, äußeren Katheterschaft 7, und am distalen Ende des letzteren ist eine im Durchmesser aufgeweitete Implantatkapsel 9 vorgesehen.

Im distalen Endbereich des ersten, inneren Katheterschafts 5 ist, in ebenfalls an sich bekannter Weise, ein Implantat-Konnektor 11 angebracht. Der Implantat-Konnektor ist mit dem ersten, inneren Katheterschaft verbunden und weißt Ösen oder Ausformungen auf, in die ein entsprechendes Gegenstück des Implantats eingreift, um das Implantat axial auf dem ersten inneren Katheterschaft solange zu fixieren, wie der Implantat-Konnektor 11 von der Implantatkapsel überdeckt ist. Die umgekehrte Variante ist ebenso zweckmäßig. Der erste, innere Katheterschaft 5 und der zweite, äußere Katheterschaft 7 sind in Längsrichtung des Einführkatheters 1 relativ zueinander verschieblich, was sowohl ein Freisetzen des - typischerweise aus einem Formgedächtnismaterial gefertigten - Stents der Herzklappenprothese 3 als auch das erwähnte Resheathen desselben per manueller Steuerung durch den Operateur vom (nicht gezeigten) proximalen Katheterende aus ermöglicht. Direkt proximal am Implantat-Konnektor 11 ist ein konisches distales Stützelement 13 am inneren Katheterschaft 5 angebracht, und am proximalen Ende der Implantatkapsel 9 ist ein proximales Stützelement 15 am äußeren Katheterschaft 7 angebracht. Während das distale Stützelement 13 zusammen mit dem inneren Katheterschaft 5 beweglich ist, ist das proximale Stützelement 15 zusammen mit dem äußeren Katheterschaft 7 beweglich. Jedes der beiden Stützelemente 13, 15 für sich allein, und in noch verbesserter Weise beide zusammen, dient/dienen zur Verhinderung eines Knickens des Einführkatheters 1 im Bereich seiner Implantatkapsel und damit verbundenen Unbrauchbarwerdens desselben bei der Rückführung der Herzklappenprothese 3 in die Implantatkapsel 9.

Bevorzugt ist das distale Stützelement 13 mit dem Implantat-Konnektor 11 verbunden. Die Verbindung kann als Schweiß-, Klebe-, Steck- oder Schraubverbindung ausgeführt werden. Prinzipiell kommt jede Verbindung in Frage. Ebenso zweckmäßig ist eine Ausführung, wo Implantat-Konnektor 11 und distales Stützelement 13 ein integrales Bauteil bilden.

Fig. 2 zeigt ein Ausführungsbeispiel des proximalen Stützelements 15, welches hier einen ersten zylindrischen Abschnitt 15a, einen zweiten zylindrischen Abschnitt 15b mit kleinerem Durchmesser und einen die beiden zylindrischen Abschnitte 15a, 15b miteinander verbindenden konischen Abschnitt 15c umfasst. Vom distalen Ende des zylindrischen Abschnitts 15b bis hin zum distalen Ende des Stützelementes 15 ist dieses durch Tangential jeweils über den weitaus größeren Teil des Umfangs verlaufende Einschnitte 15d biegeweich gestaltet.

Distales Stützelement 13 und/oder proximales Stützelement 15 können als geschnittene Edelstahlkonen ausgeführt sein. Hierbei ist es zweckmäßig, die geschnittenen Edelstahlkonen der Stützelement 13 und/oder 15 mit den geschnittenen Edelstahlkernen des ersten inneren bzw. zweiten äußeren Katheterschaftes zu verschweißen. Dabei werden vorteilhafterweise die geschnitten Edelstahlkerne bzw. Edelstahlkonen innen und/oder außen mit Kunststoff umhüllt, um ein reibungsfreieres Gleiten ineinander bzw. gegenüber der Gefäßwand zu gewährleisten. Zusätzlich wird durch die Kunststoffumhüllung eine Abdichtung gewährleistet.

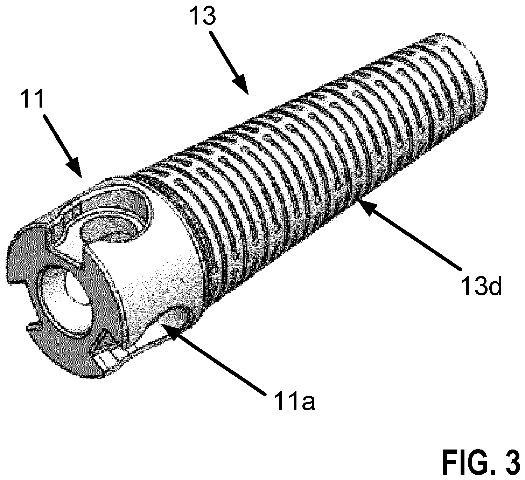

Fig. 3 zeigt eine Ausgestaltung des distalen Stützelementes 13, welches in dieser Ausgestaltung als integrales Edelstahlbauteil mit dem Implantat-Konnektor 11 ausgeführt ist. In dieser Ausgestaltung weist der Implantat-Konnektor 11 Aufnahmen 11a für ein entsprechendes Gegenstück des Implantats auf. Durch diese Aufnahmen 11a wird das Implantat axial auf dem Einführkatheter fixiert. Das distale Stützelement 13 weist ebenfalls Einschnitte 13d entlang des Umfanges auf, um die Biegeweichheit zu erhöhen.

Im Übrigen ist die Ausführung der Erfindung auch in einer Vielzahl von Abwandlungen der hier gezeigten Beispiele und weiter oben hervorgehobenen Aspekte der Erfindung möglich.

## Patentansprüche

1. Einführkatheter (1) zum Implantieren eines kardiovaskulären Implantats (3), insbesondere einer Herzklappenprothese, mit einem ersten, inneren Katheterschaft (5), welcher innerhalb von mindestens einem zweiten, äußeren Katheterschaft (7) angeordnet ist, wobei das Implantat im distalen Bereich des Einführkatheters auf dem ersten, inneren Katheterschaft angeordnet ist und wobei ein Abschnitt nahe dem distalen Ende des zweiten, äußeren Katheterschafts als Implantatkapsel (9) zur Umhüllung des Implantats während des Einführens ausgebildet ist,
**dadurch gekennzeichnet, dass**
direkt proximal vom Implantat am ersten, inneren Katheterschaft positionsfest ein biegeweiches distales Stützelement (13) mit einem im wesentlichen kegelstumpfförmigen Abschnitt angebracht ist, der sich in proximaler Richtung verjüngt, und/oder dass am proximalen Ende der Implantatkapsel in den zweiten, äußeren Katheterschaft positionsfest ein biegeweiches proximales Stützelement (15) mit einem im wesentlichen kegelstumpfförmigen Abschnitt (15c) eingefügt ist, der sich in proximaler Richtung verjüngt.

2. Einführkatheter nach Anspruch 1, wobei das proximale Stützelement (15) einen zylindrischen distalen Abschnitt (15a), dessen Durchmesser an den Innendurchmesser der Implantatkapsel (9) und den Außendurchmesser des gecrimpten Implantats (3) angepasst ist, einen zylindrischen proximalen Abschnitt (15b), dessen Außendurchmesser an denjenigen des ersten, inneren Katheterschafts angepasst ist, und einen im wesentlichen kegelstumpfförmigen mittleren Abschnitt (15c) umfasst, der einen stetigen Übergang zwischen dem distalen und proximalen zylindrischen Abschnitt bildet.

3. Einführkatheter nach Anspruch 1 oder 2, wobei das proximale Stützelement (15) als Spritzguss- oder Drehteil ausgebildet ist.

4. Einführkatheter nach einem der vorangehenden Ansprüche, wobei das proximale Stützelement (15), insbesondere mindestens im mittleren Abschnitt, eine Vielzahl von Einschnitten (15d) aufweist, die die Biegeweichheit des Stützelementes bewirken.

5. Einführkatheter nach Anspruch 4, wobei die Einschnitte (15d) sich jeweils über einen Teil, bevorzugt den größeren Teil des Umfangs des Stützelementes (15) tangential erstrecken.

6. Einführkatheter nach einem der vorangehenden Ansprüche, wobei das distale Stützelement (13) ein distales Ende, dessen Außendurchmesser an den Innendurchmesser der Implantatkapsel (9) und den Außendurchmesser des gecrimpten Implantats (3) angepasst ist, und ein proximales Ende aufweist, dessen Innendurchmesser an den Außendurchmesser des ersten, inneren Katheterschafts (5) angepasst ist.

7. Einführkatheter nach einem der vorangehenden Ansprüche, wobei das distale Stützelement (13) als massives Kunststoffteil oder Edelstahlteil mit einem Innenlumen ausgebildet ist, dessen Durchmesser an den Außendurchmesser des ersten, inneren Katheterschafts (5) angepasst ist.

8. Einführkatheter nach Anspruch 7, wobei das distal Stützelement (13), insbesondere mindestens im mittleren Abschnitt, eine Vielzahl von Einschnitten (13d) aufweist, welche sich bevorzugt jeweils über einen Teil, besonders bevorzugt den größeren Teil, des Umfangs des Stützelementes (13) tangential erstrecken.

9. Einführkatheter nach einem der vorangehenden Ansprüche, wobei das Verhältnis zwischen Länge und größtem Durchmesser des distalen und/oder des proximalen Stützelementes zwischen 2 und 7, bevorzugt zwischen 3 und 5 und insbesondere bevorzugt zwischen 3,5 und 4,5 beträgt.

10. Einführkatheter nach einem der vorangehenden Ansprüche, wobei das Verhältnis zwischen der Länge des distalen und/oder des proximalen Stützelementes (13, 15) und der Länge der Implantatkapsel (9) wischen 0,13 und 0,82, insbesondere zwischen 0,28 und 0,42, ist.

11. Einführkatheter nach Anspruch 9, wobei sowohl ein distales als auch ein proximales Stützelement (13, 15) vorgesehen ist und beide zusammen einen Anteil der Länge der Implantatkapsel (9) von mindestens von mindestens 0,25 bis 0,85, bevorzugt von 0,45 bis 0,65, stützen.

12. Einführkatheteranordnung mit einem Einführkatheter (1) nach einem der Ansprüche 1 bis 11 und einer an die distal auf diesem angeordnete Implantatkapsel angepassten kardiovaskulären Implantat (3), insbesondere einem Stent oder einer Herzklappenprothese.

13. Einführkatheteranordnung mit einem Einführkatheter (1) nach einem der vorangehenden Ansprüche, **dadurch gekennzeichnet, dass** das proximale Stützelement (15) als Hohlkörper ausgeführt ist, der derart ausgestaltet ist, dass er das distale Stützelement (13) ohne Verkeilen in sich aufnehmen kann.

14. Einführkatheteranordnung mit einem Einführkatheter (1) nach Anspruch 13, **dadurch gekennzeichnet, dass** distales und proximales Stützelement unterschiedliche Radien des Konus aufweisen, und/oder das proximale und/oder distale Stützelement ein Anschlag aufweist und/oder beide Stützelemente eine Oberflächenstruktur aufweisen, um ein Verkeilen zu verhindern.

## Claims

1. Insertion catheter (1) for implanting a cardiovascular implant (3), especially a heart valve prosthesis, with a first, inner catheter shaft (5) that is arranged within at least one second, outer catheter shaft (7), the implant being arranged on the first, inner catheter shaft in the distal area of the insertion catheter, and a section close to the distal end of the second, outer catheter shaft being in the form of an implant capsule (9) to envelop the implant during insertion,
**characterized in that**
on the first, inner catheter shaft there is, directly proximal of the implant, a positionally fixed soft-bending distal support element (13) with an essentially frustum-shaped section that tapers in the proximal direction, and/or that there is, at the proximal end of the implant capsule, a positionally fixed soft-bending proximal support element (15) inserted into the second, outer catheter shaft, this soft-bending proximal support element having an essentially frustum-shaped section (15c) that tapers in the proximal direction.

2. Insertion catheter according to claim 1, wherein the proximal support element (15) comprises a cylindrical distal section (15a), whose diameter is adapted to the inside diameter of the implant capsule (9) and to the outside diameter of the crimped implant (3), a cylindrical proximal section (15b), whose outside diameter is adapted to that of the first, inner catheter shaft, and an essentially frustum-shaped middle section (15c) that forms a continuous transition between the distal and proximal cylindrical sections.

3. Insertion catheter according to claim 1 or 2, wherein the proximal support element (15) is in the form of an injection molded or turned part.

4. Insertion catheter according to any one of the preceding claims, wherein the proximal support element (15) has, especially at least in the middle section, multiple cuts (15d) that give the support element bending flexibility.

5. Insertion catheter according to claim 4, wherein each of the cuts (15d) extends tangentially over part, preferably over most of the periphery of the support element (15).

6. Insertion catheter according to any one of the preceding claims, wherein the distal support element (13) has a distal end whose outside diameter is adapted to the inside diameter of the implant capsule (9) and to the outside diameter of the crimped implant (3), and a proximal end whose inside diameter is adapted to the outside diameter of the first, inner catheter shaft (5).

7. Insertion catheter according to any one of the preceding claims, wherein the distal support element (13) is in the form of a solid plastic part or stainless steel part with an inner lumen whose diameter is adapted to the outside diameter of the first, inner catheter shaft (5).

8. Insertion catheter according to claim 7, wherein the distal support element (13) has, especially at least in the middle section, multiple cuts (13d) each of which preferably extends tangentially over part, especially preferably over most of the periphery of the support element (13).

9. Insertion catheter according to any one of the preceding claims, wherein the ratio between the length and the largest diameter of the distal and/or proximal support element is between 2 and 7, preferably between 3 and 5, and especially preferably between 3.5 and 4.5.

10. Insertion catheter according to any one of the preceding claims, wherein the ratio between the length of the distal and/or proximal support element (13, 15) and the length of the implant capsule (9) is between 0.13 and 0.82, especially preferably between 0.28 and 0.42.

11. Insertion catheter according to claim 9, wherein both a distal and a proximal support element (13, 15) are provided and the proportion of the length of the implant capsule (9) that is supported by the two taken together is at least 0.25 to 0.85, preferably from 0.45 to 0.65.

12. An insertion catheter arrangement with an insertion catheter (1) according to any one of the claims 1 through 11 and, arranged at the distal end of this insertion catheter (1), an implant capsule adapted to the cardiovascular implant (3), this cardiovascular implant (3) being especially a stent or a heart valve prosthesis.

13. An insertion catheter arrangement with an insertion catheter (1) according to any one of the preceding claims, **characterized in that** the proximal support element (15) is in the form of a hollow body that is designed so that it can hold the distal support element (13) without wedging.

14. An insertion catheter arrangement with an insertion catheter (1) according to claim 13, **characterized in that** the distal and proximal support elements have different cone radii, and/or the proximal and/or distal support element(s) has/have a stop and/or both support elements have a surface structure, to prevent wedging.

## Revendications

1. Cathéter d'insertion (1) pour implanter un implant cardiovasculaire (3), notamment une prothèse de valve cardiaque, avec une première tige de cathéter intérieure (5), laquelle est disposée à l'intérieur d'au moins une deuxième tige de cathéter extérieure (7), où l'implant est disposé au niveau de la zone distale du cathéter d'insertion sur la première tige de cathéter intérieure, et où un segment proche de l'extrémité distale de la deuxième tige de cathéter extérieure est conçu sous la forme d'une capsule d'implant (9) pour envelopper l'implant pendant l'insertion, **caractérisé en ce que,**
directement de manière proximale par rapport à l'implant, un élément support distal (13) souple en flexion est rapporté dans une position fixe sur la première tige de cathéter intérieure avec un segment essentiellement en forme de cône tronqué, qui s'amenuise dans la direction proximale, et/ou qu'au niveau de l'extrémité proximale de la capsule d'implant, un élément support proximal (15) souple en flexion est rajouté dans une position fixe dans la deuxième tige de cathéter extérieure avec un segment essentiellement en forme de cône tronqué (15c) qui s'amenuise dans la direction proximale.

2. Cathéter d'insertion selon la revendication 1, dans lequel l'élément support proximal (15) comprend un segment distal (15a) cylindrique dont le diamètre est ajusté au diamètre intérieur de la capsule d'implant (9) et au diamètre extérieur de l'implant (3) serti, un segment proximal (15b) cylindrique dont le diamètre extérieur est ajusté à celui de la première tige de cathéter intérieure, et un segment central (15c) essentiellement en forme de cône tronqué qui forme une transition régulière entre les segments cylindriques distal et proximal.

3. Cathéter d'insertion selon la revendication 1 ou 2, dans lequel l'élément support proximal (15) est conçu sous la forme d'une pièce moulée par injection ou d'une pièce rotative.

4. Cathéter d'insertion selon l'une des revendications précédentes, dans lequel l'élément support proximal (15) présente une multiplicité de découpes (15d), notamment, au moins dans le segment central, qui assurent la souplesse en flexion de l'élément support.

5. Cathéter d'insertion selon la revendication 4, dans lequel les découpes (15d) s'étendent tangentiellement respectivement sur une partie, de préférence, la plus grande partie du périmètre de l'élément support (15).

6. Cathéter d'insertion selon l'une des revendications précédentes, dans lequel l'élément support distal (13) présente une extrémité distale dont le diamètre extérieur est ajusté au diamètre intérieur de la capsule d'implant (9) et au diamètre extérieur de l'implant (3) serti, et une extrémité proximale, dont le diamètre intérieur est ajusté au diamètre extérieur de la première tige de cathéter intérieure (5).

7. Cathéter d'insertion selon l'une des revendications précédentes, dans lequel l'élément support distal (13) est conçu sous la forme d'une pièce en matière plastique ou en acier allié massive avec une lumière intérieure, dont le diamètre est ajusté au diamètre extérieur de la première tige de cathéter intérieure (5).

8. Cathéter d'insertion selon la revendication 7, dans lequel l'élément support distal (13), notamment, au moins dans le segment central, présente une multiplicité de découpes (13d), lesquelles s'étendent de manière tangentielle de préférence respectivement sur une partie, de manière particulièrement préférée, sur la plus grande partie du périmètre de l'élément d'appui (13).

9. Cathéter d'insertion selon l'une des revendications précédentes, dans lequel le rapport entre la longueur et le diamètre le plus grand de l'élément support distal et/ou proximal se situe entre 2 et 7, de préférence entre 3 et 5 et de manière particulièrement préférée entre 3,5 et 4,5.

10. Cathéter d'insertion selon l'une des revendications précédentes, dans lequel le rapport entre la longueur des éléments supports (13, 15) distal et/ou proximal et la longueur de la capsule d'implant (9) se situe entre 0,13 et 0,82, de préférence entre 0,28 et 0,42.

11. Cathéter d'insertion selon la revendication 9, dans lequel à la fois un élément support distal et un élément support proximal (13, 15) sont prévus et les deux portent ensemble une proportion en longueur de la capsule d'implant (9) d'au moins 0,25 à 0,85, de préférence de 0,45 à 0,65.

12. Agencement de cathéter d'insertion doté d'un cathéter d'insertion (1) selon l'une des revendications 1 à 11 et d'une capsule d'implant disposée de manière distale sur celui-ci, ajustée à l'implant cardiovasculaire (3), notamment un stent ou une prothèse de valve cardiaque.

13. Agencement de cathéter d'insertion doté d'un cathéter d'insertion (1) selon l'une des revendications précédentes, **caractérisé en ce que** l'élément support proximal (15) est conçu sous la forme d'un corps creux qui est dessiné de telle manière qu'il peut recevoir l'élément support distal (13) sans pose de cales.

14. Agencement de cathéter d'insertion doté d'un cathéter d'insertion (1) selon la revendication 13, **caractérisé en ce que** les éléments supports distal et proximal présentent des rayons de cônes différents, et/ou que l'éléments proximal et/ou l'élément distal présente une butée, et/ou les deux éléments supports présentent une structure de surface, afin d'éviter une pose de cales.
